# EUROPEAN PATENT APPLICATION

(11) **EP 2 927 211 A1**
(43) Date of publication of application: **07.10.2015**
(21) Application number: 13858294.5
(22) Date of filing: 29.11.2013
(51) Int. Cl.: C07C 233/09, C07C 233/02

(54) **NOVEL PSEUDOCERAMIDE COMPOUND AND PRODUCTION METHOD FOR SAME**

(30) Priority: 30.11.2012 KR 20120137853
(71) Applicant: Amorepacific Corporation, Seoul 140-777 (KR)
(72) Inventor: WOO, Byoung Young, Yongin-si Gyeonggi-do 446-729 (KR); JANG, Won Hee, Yongin-si Gyeonggi-do 446-729 (KR); JOO, Yung Hyup, Yongin-si Gyeonggi-do 446-729 (KR); PARK, Yang Hui, Yongin-si Gyeonggi-do 446-729 (KR); YI, Chang Geun, Yongin-si Gyeonggi-do 446-729 (KR); CHO, Young Suk, Yongin-si Gyeonggi-do 446-729 (KR); BAEK, Heung Soo, Yongin-si Gyeonggi-do 446-729 (KR); SHIN, Song Seok, Yongin-si Gyeonggi-do 446-729 (KR); PARK, Young Ho, Yongin-si Gyeonggi-do 446-729 (KR)
(74) Representative: Novagraaf Technologies
(86) International application number: PCT/KR2013/011026
(87) International publication number: WO 2014/084676

(57) **Abstract**

The present invention provides: a novel pseudoceramide compound, an isomer thereof, a pharmaceutically acceptable salt thereof, a prodrug thereof, a hydrate thereof or a solvate thereof. The present invention has a skin protecting effect in that the invention has a superior effect in skin moisturisation and is outstandingly stable and soluble, and thus the invention can protect the skin from external irritation without side effects, and can be used as an active substance in damaged skin recovery and prevention. Consequently, the invention can be used as a skin-moisturising dermatological external composition, cosmetic composition or pharmaceutical composition.

## Description

### [Technical Field]

The present disclosure relates to a novel pseudo-ceramide compound and a method for preparing same. More particularly, the present disclosure relates to a novel pseudo-ceramide compound as a tris(hydroxymethyl)aminomethane derivative, an isomer thereof, a pharmaceutically acceptable salt thereof, a prodrug thereof, a hydrate thereof or a solvate thereof and a method for preparing same.

### [Background Art]

Ceramides are one of the main components of the intercellular lipids which constitute the stratum corneum of the skin and serve to prevent excessive water loss due to evaporation and maintain the structure of the stratum corneum. The stratum corneum serves as a barrier to protect underlying tissue from harmful substances or microorganisms from the external environment. When differentiated keratinocytes shed from the stratum corneum, the intercellular lipids of the stratum corneum form a lamellar structure, thereby contributing to maintenance of the skin's basic function. The intercellular lipids consist of ceramide, cholesterol, free fatty acid, etc. and, among them, ceramide is known to play a key role in water retention and barrier function of the stratum corneum. A decreased ceramide content in the stratum corneum is known to result in increased evaporation of water and aggravation of various skin diseases. Also, it is known that the skin with decreased ceramide content in the stratum corneum caused by skin aging or external stimulation can be recovered to normal state by supplementing ceramide from outside. Accordingly, studies have been conducted on various natural animal and plant products containing ceramides. However, natural ceramide is difficult to be produced in large scale because of difficulty in extraction, etc. and is inappropriate for commercialization due to high cost. In addition, natural ceramide is limited in exhibiting efficacy since its content in cosmetic products is restricted because of low solubility in various solvents. Therefore, research and development of pseudo-ceramides mimicking naturally occurring ceramide and having improved physical properties, which can resolve the disadvantage of natural ceramide and can be commercialized, is necessary.

### [References of the Related Art]

### [Patent Documents]

Korean Patent Publication No. 10-2010-0001374

### [Disclosure]

### [Technical Problem]

The present disclosure is directed to providing a novel pseudo-ceramide compound having improved stability, solubility and skin moisturizing ability, wherein a fatty acid is introduced to tris(hydroxymethyl)aminomethane, and a method for preparing same.

### [Technical Solution]

In a general aspect, there is provided a novel pseudo-ceramide compound represented by Chemical Formula 1, an isomer thereof, a pharmaceutically acceptable salt thereof, a prodrug thereof, a hydrate thereof or a solvate thereof: 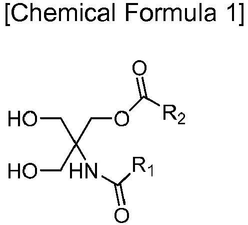 wherein each of R₁ and R₂ is independently a C₉-C₂₃ saturated or unsaturated aliphatic chain unsubstituted or substituted with a hydroxyl group.

In an exemplary embodiment of the present disclosure, each of R₁ and R₂ may be independently a C₁₁-C₁₇ saturated or unsaturated aliphatic chain unsubstituted or substituted with a hydroxyl group.

In an exemplary embodiment of the present disclosure, each of R₁ and R₂ may be independently selected from a group consisting of C₁₁H₂₃, C₁₃H₂₇, C₁₅H₃₁, C₁₇H₃₅, C₁₇H₃₁ and C₁₇H₃₃.

In an exemplary embodiment of the present disclosure, the pseudo-ceramide compound, isomer thereof, pharmaceutically acceptable salt thereof, prodrug thereof, hydrate thereof or solvate thereof may be tris(hydroxymethyl)aminomethane substituted with a fatty acid.

In an exemplary embodiment of the present disclosure, the pseudo-ceramide compound, isomer thereof, pharmaceutically acceptable salt thereof, prodrug thereof, hydrate thereof or solvate thereof may be selected from a group consisting of hexadecanoic acid 2-hexadecanoylamino-3-hydroxy-hydroxymethyl-propyl ester, dodecanoic acid 2-dodecanoylamino-3-hydroxy-2-hydroxymethyl-propyl ester, tetradecanoic acid 3-hydroxy-2-hydroxymethyl-2-tetradecanoylamino-propyl ester, octadecanoic acid 3-hydroxy-2-hydroxymethyl-2-octadecanoylamino-propyl ester, octadecanoic acid 2-dodecanoylamino-3-hydroxy-2-hydroxymethyl-propyl ester, octadecanoic acid 3-hydroxy-2-hydroxymethyl-2-tetradecanoylamino-propyl ester, tetradecanoic acid 3-hydroxy-2-hydroxymethyl-2-octadecanoylamino-propyl ester, dodecanoic acid 3-hydroxy-2-hydroxymethyl-2-octadecanoylamino-propyl ester, octadec-9-enoic acid 3-hydroxy-2-hydroxymethyl-2-octadec-9-enoylamino-propyl ester, octadeca-9,12-dienoic acid 3-hydroxy-2-hydroxymethyl-2-octadeca-9,12-dienoylamino-propyl ester, 10-hydroxy-dec-2-enoic acid 3-hydroxy-2-(10-hydroxy-dec-2-enoylamino)-2-hydroxymethyl-propyl ester, 10-hydroxy-octadecanoic acid 3-hydroxy-2-hydroxymethyl-2-(10-hydroxy-octadecanoylamino)-propyl ester, 12-hydroxy-octadecanoic acid 3-hydroxy-2-hydroxymethyl-2-(12-hydroxy-octadecanoylamino)-propyl ester and 16-hydroxy-hexadecanoic acid 3-hydroxy-2-(16-hydroxy-hexadecanoylamino)-2-hydroxymethyl-propyl ester.

In another general aspect, there is provided a method for preparing the novel pseudo-ceramide compound, isomer thereof, pharmaceutically acceptable salt thereof, prodrug thereof, hydrate thereof or solvate thereof, including reacting tris(hydroxymethyl)aminomethane and a fatty acid compound under a basic condition to synthesize a pseudo-ceramide compound.

In an exemplary embodiment of the present disclosure, the method may be represented by Scheme 1: 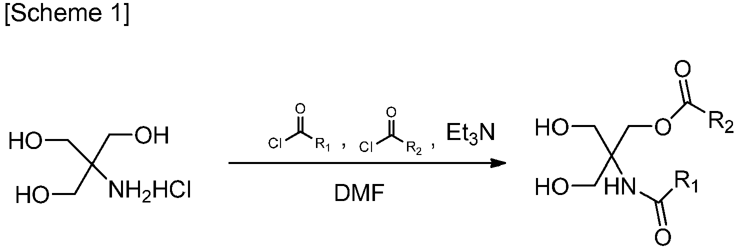 wherein each of R₁ and R₂ is independently a C₉-C₂₃ saturated or unsaturated aliphatic chain unsubstituted or substituted with a hydroxyl group.

In another general aspect, there is provided a composition for moisturizing skin, including the novel pseudo-ceramide compound, isomer thereof, pharmaceutically acceptable salt thereof, prodrug thereof, hydrate thereof or solvate thereof as an active ingredient.

In an exemplary embodiment of the present disclosure, the active ingredient may be included in an amount of 0.01-20 wt% based on the total weight of the composition.

In an exemplary embodiment of the present disclosure, the composition may be a composition for external application to skin.

In an exemplary embodiment of the present disclosure, composition may be a cosmetic composition or a pharmaceutical composition.

### [Advantageous Effects]

Since the novel pseudo-ceramide compound of the present disclosure is superior in stability, solubility and skin moisturizing effect, it can be used as an active ingredient for protecting the skin from external stimulation and recovering or preventing damage to the skin without side effects. Accordingly, it can be used in a composition for external application to skin, a cosmetic composition or a pharmaceutical composition for moisturizing the skin.

### [Description of Drawings]

Fig. 1 shows change in transepithelial electrical resistance upon treatment with a pseudo-ceramide compound according to an exemplary embodiment of the present disclosure.
Fig. 2 shows a quantitative analysis result of ceramides in artificial skin upon treatment with a pseudo-ceramide compound according to an exemplary embodiment of the present disclosure.

### [Best Mode]

Hereinafter, exemplary embodiments of the present disclosure will be described in detail so that those of ordinary skill in the art to which the present disclosure belongs can easily carry out the present disclosure.

As used herein, "skin" means the tissue covering the body surface of an animal and is used in the broadest sense, including not only the tissue covering the surface of face or body but also the scalp and hair.

As used herein, "pharmaceutically acceptable" means being devoid of substantial toxic effects when used in a usually employed medicinal dosage and thereby being approvable or approved by the government or an international organization comparable thereto for use in animals, and more particularly in humans, or being listed in the pharmacopeia.

As used herein, "pharmaceutically acceptable salt" refers to a salt according to an aspect of the present disclosure that is pharmaceutically acceptable and possesses the desired pharmacological activity of the parent compound. Such salts include: (1) acid addition salts formed with inorganic acids such as hydrochloric acid, hydrobromic acid, sulfuric acid, nitric acid, phosphoric acid, etc. or formed with organic acids such as acetic acid, propionic acid, hexanoic acid, cyclopentylpropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid and muconic acid; or (2) salts formed when an acidic proton present in the parent compound is replaced.

As used herein, "prodrug" refers to a drug whose physical and chemical properties have been changed chemically such that it does not exhibit a physiological activity as it is but is converted to an active drug through chemical or enzymatic processes after being administered.

As used herein, "hydrate" refers to a compound to which water is bound. The binding between water and the compound includes non-covalent binding.

As used herein, "solvate" refers to a complex formed by a solute molecule or ion and a solvent molecule or ion.

As used herein, "isomers" refers to compounds of the present disclosure or salts thereof with the same chemical formula or molecular formula but different optical or steric properties.

Unless specified otherwise, the term "compound according to the present disclosure" or "compound represented by Chemical Formula 1" includes the compound itself, a pharmaceutically acceptable salt thereof, a hydrate thereof, a solvate thereof, an isomer thereof and a prodrug thereof.

The present disclosure provides a novel pseudo-ceramide compound represented by Chemical Formula 1, an isomer thereof, a pharmaceutically acceptable salt thereof, a prodrug thereof, a hydrate thereof or a solvate thereof: 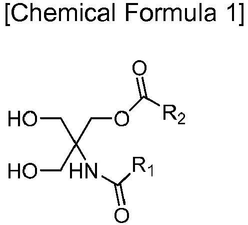 wherein each of R₁ and R₂ is independently a C₉-C₂₃ saturated or unsaturated aliphatic chain unsubstituted or substituted with a hydroxyl group.

Each of R₁ and R₂ is a C₉-C₂₃ saturated or unsaturated aliphatic chain or a C₉-C₂₃ saturated or unsaturated aliphatic chain having a hydroxyl group. R₁ and R₂ may be the same or different from each other.

Specifically, each of R₁ and R₂ may be independently a C₁₁-C₁₇ saturated or unsaturated aliphatic chain. More specifically, each of R₁ and R₂ may be independently selected from a group consisting of a C₁₁H₂₃, C₁₃H₂₇, C₁₅H₃₁ or C₁₇H₃₅ saturated aliphatic chain and a C₁₇H₃₁ or C₁₇H₃₃ unsaturated aliphatic chain.

The pseudo-ceramide compound according to the present disclosure has an amide moiety and an ester moiety in one molecule and has various fatty acid derivatives substituted therein. It may be tris(hydroxymethyl)aminomethane substituted with a saturated or unsaturated fatty acid.

The pseudo-ceramide compound according to the present disclosure may be prepared by a method including reacting tris(hydroxymethyl)aminomethane and a fatty acid compound under a basic condition using triethylamine to synthesize a pseudo-ceramide compound.

A method for preparing the pseudo-ceramide compound according to the present disclosure may be schematically represented by Scheme 1. That is to say, tris(hydroxymethyl)aminomethane may be reacted with 1 equivalent of a fatty acid compound having R₁ and 1 equivalent of a fatty acid compound having R₂ to synthesize the pseudo-ceramide compound represented by Chemical Formula 1.

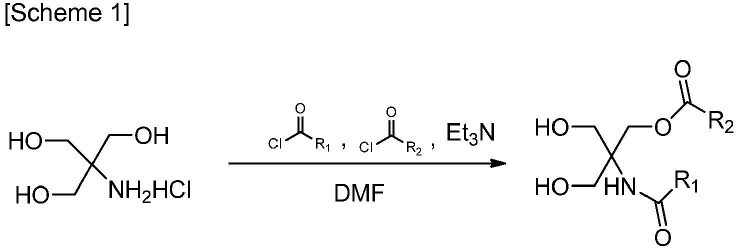

In Scheme 1, each of R₁ and R₂ is independently a C₉-C₂₃ saturated or unsaturated aliphatic chain unsubstituted or substituted with a hydroxyl group.

The pseudo-ceramide compound according to the present disclosure may be selected from a group consisting of hexadecanoic acid 2-hexadecanoylamino-3-hydroxy-hydroxymethyl-propyl ester, dodecanoic acid 2-dodecanoylamino-3-hydroxy-2-hydroxymethyl-propyl ester, tetradecanoic acid 3-hydroxy-2-hydroxymethyl-2-tetradecanoylamino-propyl ester, octadecanoic acid 3-hydroxy-2-hydroxymethyl-2-octadecanoylamino-propyl ester, octadecanoic acid 2-dodecanoylamino-3-hydroxy-2-hydroxymethyl-propyl ester, octadecanoic acid 3-hydroxy-2-hydroxymethyl-2-tetradecanoylamino-propyl ester, tetradecanoic acid 3-hydroxy-2-hydroxymethyl-2-octadecanoylamino-propyl ester, dodecanoic acid 3-hydroxy-2-hydroxymethyl-2-octadecanoylamino-propyl ester, octadec-9-enoic acid 3-hydroxy-2-hydroxymethyl-2-octadec-9-enoylamino-propyl ester, octadeca-9,12-dienoic acid 3-hydroxy-2-hydroxymethyl-2-octadeca-9,12-dienoylamino-propyl ester, 10-hydroxy-dec-2-enoic acid 3-hydroxy-2-(10-hydroxy-dec-2-enoylamino)-2-hydroxymethyl-propyl ester, 10-hydroxy-octadecanoic acid 3-hydroxy-2-hydroxymethyl-2-(10-hydroxy-octadecanoylamino)-propyl ester, 12-hydroxy-octadecanoic acid 3-hydroxy-2-hydroxymethyl-2-(12-hydroxy-octadecanoylamino)-propyl ester and 16-hydroxy-hexadecanoic acid 3-hydroxy-2-(16-hydroxy-hexadecanoylamino)-2-hydroxymethyl-propyl ester, although not particularly being limited thereto.

The pseudo-ceramide compound according to the present disclosure exhibits excellent skin moisturizing effect while having superior stability and solubility.

Accordingly, the present disclosure provides a composition for moisturizing skin, including the pseudo-ceramide compound, an isomer thereof, a pharmaceutically acceptable salt thereof, a prodrug thereof, a hydrate thereof or a solvate thereof as an active ingredient.

In the composition of the present disclosure, the active ingredient may be included in an amount of 0.01-20 wt%, specifically 0.1-10 wt%, more specifically 0.5-5 wt%, based on the total weight of the composition. When the active ingredient is included in an amount in the above-described range, the effect desired by the present disclosure can be adequately achieved while ensuring both stability and solubility of the composition and providing good cost effectiveness. Specifically, if the content of the pseudo-ceramide compound, an isomer thereof, a pharmaceutically acceptable salt thereof, a prodrug thereof, a hydrate thereof or a solvate thereof is less than 0.01 wt%, a sufficient skin moisturizing effect may not be achieved. And, if it exceeds 20 wt%, cost effectiveness may be unsatisfactory.

The present disclosure also provides a composition for external application to skin, including the pseudo-ceramide compound, an isomer thereof, a pharmaceutically acceptable salt thereof, a prodrug thereof, a hydrate thereof or a solvate thereof as an active ingredient.

The present disclosure also provides a cosmetic composition, including the pseudo-ceramide compound, an isomer thereof, a pharmaceutically acceptable salt thereof, a prodrug thereof, a hydrate thereof or a solvate thereof as an active ingredient.

The cosmetic composition according to the present disclosure may be provided in the form of any formulation suitable for topical application. For example, it may be provided in the form of solution, oil-in-water emulsion, water-in-oil emulsion, suspension, solid, gel, powder, paste, foam or aerosol. These formulations can be prepared according to the methods commonly employed in the art.

The cosmetic composition according to the present disclosure may further include other ingredients providing synergic effect without negatively affecting the desired effect. Specifically, the cosmetic composition according to the present disclosure may further include arbutin and ascorbic acid derivatives which may provide skin whitening effect. In addition, the cosmetic composition according to the present disclosure may further include a moisturizing agent, an emollient agent, a surfactant, a UV absorbent, an antiseptic, a sterilizer, an antioxidant, a pH adjusting agent, an organic or inorganic pigment, a flavor, a cooling agent or an antiperspirant. The contents of those ingredients may be easily determined by those skilled in the art within the ranges not deteriorating the purpose and effect of the present disclosure. They may be included in an amount of 0.01-5 wt%, specifically 0.01-3 wt%, based on the total weight of the composition.

The present disclosure also provides a pharmaceutical composition including the compound, an isomer thereof, a pharmaceutically acceptable salt thereof, a prodrug thereof, a hydrate thereof or a solvate thereof as an active ingredient. The pharmaceutical composition may exhibit excellent skin moisturizing effect.

The pharmaceutical composition according to the present disclosure may be administered orally or parenterally, e.g., rectally, topically, transdermally, intravenously, intramuscularly, intraperitoneally, subcutaneously, etc. Formulations for oral administration may include tablet, pill, soft or hard capsule, granule, powder, fine granule, liquid, emulsion or pellet, although not being limited thereto. Formulations for parenteral administration may include solution, suspension, emulsion, gel, injectable solution, drip, suppository, patch or spray, although not being limited thereto. These formulations may be prepared easily according to the methods commonly employed in the art and may include a surfactant, an excipient, a hydrating agent, an emulsifier, a suspending agent, a salt or buffer for adjusting osmotic pressure, a coloring agent, a flavor, a stabilizer, an antiseptic, a preservative or other commonly used adjuvants, if desired.

Determination of the administration dose of the active ingredient is within the level of those skilled in the art. Although a daily administration dose may be varied with the severity and stage of the condition to be treated, age and physical condition of a subject to be treated, presence of complication(s), or the like, the composition may be administered with a daily dose of 1 µg/kg to 200 mg/kg, specifically 50 µg/kg to 50 mg/kg, once to three times a day. However, the scope of the present disclosure is not limited by the above administration dose by any means.

Hereinafter, the present disclosure will be described in detail through examples. However, the following examples are for illustrative purposes only and it will be apparent to those of ordinary skill in the art that the scope of the present disclosure is not limited by the examples.

### [Example 1] Preparation of hexadecanoic acid 2-hexadecanoylamino-3-hydroxy-hydroxymethyl-propyl ester

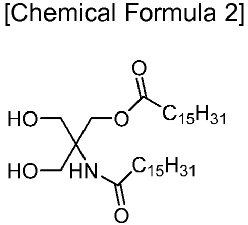

Tris(hydroxymethyl)aminomethane (10 g, 1 eq) was dissolved in dimethylformaldehyde (100 mL). After adding triethylamine (23 mL, 2 eq), palmitoyl chloride (22.6 g, 1 eq) was slowly added dropwise for 30 minutes while stirring at room temperature. After stirring for 2 hours at room temperature, palmitoyl chloride (22.6 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 30 g of white solid (60%) was obtained using a silica column. The obtained white solid was hexadecanoic acid 2-hexadecanoylamino-3-hydroxy-hydroxymethyl-propyl ester represented by Chemical Formula 2. ¹H NMR analysis result of the white solid is as follows.

¹H NMR (300 MHz, DMSO-d₆) 6.23 (br, 1H), 4.29 (s, 2H), 4.22 (t, J = 6.6 Hz, 2H), 3.70-3.64 (m, 2H), 3.54-3.48 (m, 2H), 2.36 (t, J = 7.5 Hz, 2H), 2.22 (t, J = 7.5 Hz, 2H), 1.62-1.60 (m, 4H), 1.42-1.11 (m, 48H), 0.90-0.85 (m, 6H).

### [Example 2] Preparation of dodecanoic acid 2-dodecanoylamino-3-hydroxy-2-hydroxymethyl-propyl ester

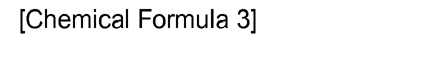

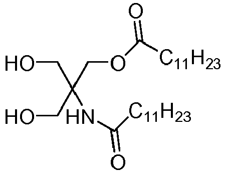

Tris(hydroxymethyl)aminomethane (10 g, 1 eq) was dissolved in dimethylformaldehyde (100 mL). After adding triethylamine (23 mL, 2 eq), lauroyl chloride (18.0 g, 1 eq) was slowly added dropwise for 30 minutes while stirring at room temperature. After stirring for 2 hours at room temperature, lauroyl chloride (18.0 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 26 g of white solid (65%) was obtained using a silica column. The obtained white solid was dodecanoic acid 2-dodecanoylamino-3-hydroxy-2-hydroxymethyl-propyl ester represented by Chemical Formula 3. ¹H NMR analysis result of the white solid is as follows.

¹H NMR (300 MHz, DMSO-d₆) 6.21 (s, 1H), 4.29 (s, 2H), 4.18-4.14 (m, 2H), 3.68-3.64 (m, 2H), 3.52-3.48 (m, 2H), 2.36 (t, J = 7.5 Hz, 2H), 2.22 (t, J = 7.5 Hz, 2H), 1.62-1.60 (m, 4H), 1.42-1.11 (m, 32H), 0.90-0.85 (m, 6H).

### [Example 3] Preparation of tetradecanoic acid 3-hydroxy-2-hydroxymethyl-2-tetradecanoylamino-propyl ester

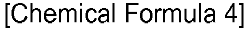

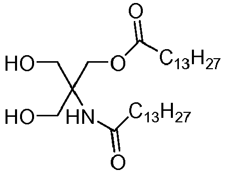

Tris(hydroxymethyl)aminomethane (10 g, 1 eq) was dissolved in dimethylformaldehyde (100 mL). After adding triethylamine (23 mL, 2 eq), myristoyl chloride (20.3 g, 1 eq) was slowly added dropwise for 30 minutes while stirring at room temperature. After stirring for 2 hours at room temperature, myristoyl chloride (20.3 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 38 g of white solid (63%) was obtained using a silica column. The obtained white solid was tetradecanoic acid 3-hydroxy-2-hydroxymethyl-2-tetradecanoylamino-propyl ester represented by Chemical Formula 4. ¹H NMR analysis result of the white solid is as follows.

¹H NMR (300 MHz, CDCl₃) 6.21 (s, 1H), 4.29 (s, 2H), 4.18-4.14 (m, 2H), 3.68-3.64 (m, 2H), 3.52-3.49 (m, 2H), 2.36 (t, J = 7.5 Hz, 2H), 2.22 (t, J = 7.5 Hz, 2H), 1.62-1.60 (m, 4H), 1.42-1.11 (m, 40H), 0.90-0.86 (m, 6H).

### [Example 4] Preparation of octadecanoic acid 3-hydroxy-2-hydroxymethyl-2-octadecanoylamino-propyl ester

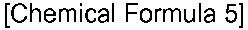

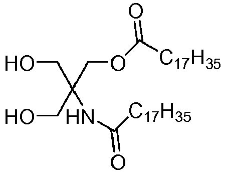

Tris(hydroxymethyl)aminomethane (10 g, 1 eq) was dissolved in dimethylformaldehyde (100 mL). After adding triethylamine (23 mL, 2 eq), stearoyl chloride (25 g, 1 eq) was slowly added dropwise for 30 minutes while stirring at room temperature. After stirring for 2 hours at room temperature, stearoyl chloride (25 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 20 g of white solid (37%) was obtained using a silica column. The obtained white solid was tetradecanoic acid 3-hydroxy-2-hydroxymethyl-2-tetradecanolyamino-propyl ester represented by Chemical Formula 5. ¹H NMR analysis result of the white solid is as follows.

¹H NMR (300 MHz, CDCl₃) 6.21 (s, 1H), 4.29 (s, 2H), 4.18-4.14 (m, 2H), 3.69-3.65 (m, 2H), 3.53-3.49 (m, 2H), 2.36 (t, J = 7.5 Hz, 2H), 2.22 (t, J = 7.5 Hz, 2H), 1.62-1.60 (m, 4H), 1.42-1.11 (m, 56H), 0.90-0.86 (m, 6H).

### [Example 5] Preparation of octadecanoic acid 2-dodecanoylamino-3-hydroxy-2-hydroxymethyl-propyl ester

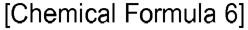

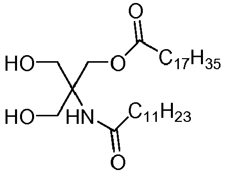

The obtained white solid was tetradecanoic acid 3-hydroxy-2-hydroxymethyl-2-tetradecanolyamino-propyl ester represented by Chemical Formula 3. ¹H NMR analysis result of the white solid is as follows.

The obtained white solid was octadecanoic acid 2-dodecanoylamino-3-hydroxy-2-hydroxymethyl-propyl ester represented by Chemical Formula 6. ¹H NMR analysis result of the white solid is as follows.

¹H NMR (300 MHz, DMSO-d₆) 6.21 (s, 1H), 4.29 (s, 2H), 4.18-4.14 (m, 2H), 3.69-3.65 (m, 2H), 3.53-3.50 (m, 2H), 2.36 (t, J = 7.5 Hz, 2H), 2.22 (t, J = 7.5 Hz, 2H), 1.62-1.60 (m, 4H), 1.42-1.11 (m, 44H), 0.90-0.86 (m, 6H).

### [Example 6] Preparation of octadecanoic acid 3-hydroxy-2-hydroxymethyl-2-tetradecanoylamino-propyl ester

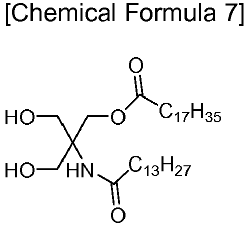

Tris(hydroxymethyl)aminomethane (10 g, 1 eq) was dissolved in dimethylformaldehyde (100 mL). After adding triethylamine (23 mL, 2 eq), myristoyl chloride (21 g, 1 eq) was slowly added dropwise for 30 minutes while stirring at room temperature. After stirring for 2 hours at room temperature, stearoyl chloride (25 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 20 g of white solid (40%) was obtained using a silica column. The obtained white solid was octadecanoic acid 3-hydroxy-2-hydroxymethyl-2-tetradecanoylamino-propyl ester represented by Chemical Formula 7. ¹H NMR analysis result of the white solid is as follows.

¹H NMR (300 MHz, CDCl₃) 6.22 (s, 1H), 4.29 (s, 2H), 4.18-4.14 (m, 2H), 3.69-3.65 (m, 2H), 3.53-3.49 (m, 2H), 2.37 (t, J = 7.5 Hz, 2H), 2.22 (t, J = 7.5 Hz, 2H), 1.62-1.60 (m, 4H), 1.42-1.11 (m, 48H), 0.90-0.86 (m, 6H).

### [Example 7] Preparation of tetradecanoic acid 3-hydroxy-2-hydroxymethyl-2-octadecanoylamino-propyl ester

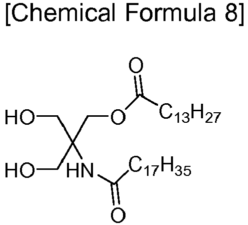

Tris(hydroxymethyl)aminomethane (10 g, 1 eq) was dissolved in dimethylformaldehyde (100 mL). After adding triethylamine (23 mL, 2 eq), stearoyl chloride (25 g, 1 eq) was slowly added dropwise for 30 minutes while stirring at room temperature. After stirring for 2 hours at room temperature, myristoyl chloride (21 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 22 g of white solid (45%) was obtained using a silica column. The obtained white solid was tetradecanoic acid 3-hydroxy-2-hydroxymethyl-2-octadecanoylamino-propyl ester represented by Chemical Formula 8. ¹H NMR analysis result of the white solid is as follows.

¹H NMR (300 MHz, CDCl₃) 6.21 (s, 1H), 4.29 (s, 2H), 4.18-4.14 (m, 2H), 3.69-3.65 (m, 2H), 3.53-3.49 (m, 2H), 2.37 (t, J = 7.5 Hz, 2H), 2.22 (t, J = 7.5 Hz, 2H), 1.62-1.60 (m, 4H), 1.42-1.11 (m, 48H), 0.90-0.86 (m, 6H).

### [Example 8] Preparation of dodecanoic acid 3-hydroxy-2-hydroxymethyl-2-octadecanoylamino-propyl ester

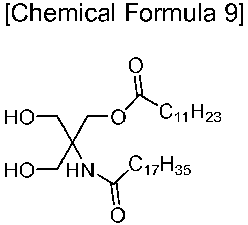

Tris(hydroxymethyl)aminomethane (10 g, 1 eq) was dissolved in dimethylformaldehyde (100 mL). After adding triethylamine (23 mL, 2 eq), stearoyl chloride (25 g, 1 eq) was slowly added dropwise for 30 minutes while stirring at room temperature. After stirring for 2 hours at room temperature, lauroyl chloride (18 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 24 g of white solid (51%) was obtained using a silica column. The obtained white solid was dodecanoic acid 3-hydroxy-2-hydroxymethyl-2-octadecanoylamino-propyl ester represented by Chemical Formula 9. ¹H NMR analysis result of the white solid is as follows.

¹H NMR (300 MHz, CDCl₃) 6.21 (s, 1H), 4.29 (s, 2H), 4.18-4.14 (m, 2H), 3.69-3.65 (m, 2H), 3.53-3.49 (m, 2H), 2.37 (t, J = 7.5 Hz, 2H), 2.22 (t, J = 7.5 Hz, 2H), 1.62-1.60 (m, 4H), 1.42-1.11 (m, 44H), 0.90-0.86 (m, 6H).

### [Example 9] Preparation of octadec-9-enoic acid 3-hydroxy-2-hydroxymethyl-2-octadec-9-enoylamino-propyl ester

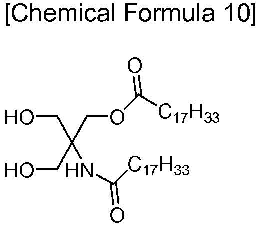

Tris(hydroxymethyl)aminomethane (10 g, 1 eq) was dissolved in dimethylformaldehyde (100 mL). After adding triethylamine (23 mL, 2 eq), oleyl chloride (25 g, 1 eq) was slowly added dropwise for 30 minutes while stirring at room temperature. After stirring for 2 hours at room temperature, oleyl chloride (25 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 21 g of white solid (39%) was obtained using a silica column. The obtained white solid was octadec-9-enoic acid 3-hydroxy-2-hydroxymethyl-2-octadec-9-enoylamino-propyl ester represented by Chemical Formula 10. ¹H NMR analysis result of the white solid is as follows.

¹H NMR (300 MHz, CDCl₃) 6.21 (s, 1H), 5.42-5.34 (m, 4H), 4.29 (s, 2H), 4.18-4.14 (m, 2H), 3.69-3.65 (m, 2H), 3.53-3.49 (m, 2H), 2.40-1.90 (m, 12H), 1.62-1.60 (m, 4H), 1.42-1.11 (m, 38H), 0.90-0.86 (m, 6H).

### [Example 10] Preparation of octadeca-9,12-dienoic acid 3-hydroxy-2-hydroxymethyl-2-octadeca-9,12-dienoylamino-propyl ester

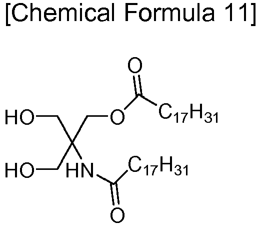

Tris(hydroxymethyl)aminomethane (10 g, 1 eq) was dissolved in dimethylformaldehyde (100 mL). After adding triethylamine (23 mL, 2 eq), linoleyl chloride (25 g, 1 eq) was slowly added dropwise for 30 minutes while stirring at room temperature. After stirring for 2 hours at room temperature, linoleyl chloride (25 g, 1 eq) was slowly added dropwise for 30 minutes and the mixture was further stirred for 6 hours. Upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 18 g of white solid (33%) was obtained using a silica column. The obtained white solid was octadeca-9,12-dienoic acid 3-hydroxy-2-hydroxymethyl-2-octadeca-9,12-dienoylamino-propyl ester represented by Chemical Formula 11. ¹H NMR analysis result of the white solid is as follows.

¹H NMR (300 MHz, CDCl₃) 6.21 (s, 1H), 5.50-5.20 (m, 8H), 4.29 (s, 2H), 4.18-4.14 (m, 2H), 3.69-3.65 (m, 2H), 3.53-3.49 (m, 2H),2.90-2.70 (m, 4H), 2.40-1.90 (m, 12H), 1.62-1.60 (m, 4H), 1.42-1.11 (m, 28H), 0.90-0.86 (m, 6H).

### [Example 11] Preparation of 10-hydroxy-dec-2-enoic acid 3-hydroxy-2-(10-hydroxy-dec-2-enoylamino)-2-hydroxymethyl-propyl ester

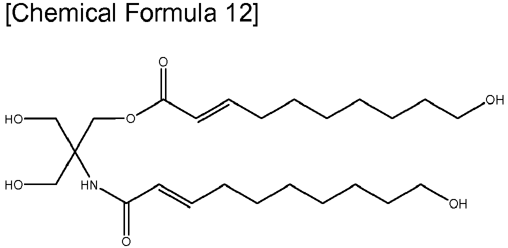

Tris(hydroxymethyl)aminomethane (10 g, 1 eq) was dissolved in dimethylformaldehyde (100 mL). After adding dicyclohexylcarbodiimide (34 g, 2 eq) and 4-dimethylaminopyridine (4.03 g, 0.2 eq), 10-hydroxy-2-decanoic acid (30.7 g, 2 eq) was slowly added dropwise for 30 minutes while stirring at room temperature. After stirring for 6 hours, upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 15 g of white solid (39%) was obtained using a silica column. The obtained white solid was 10-hydroxy-dec-2-enoic acid 3-hydroxy-2-(10-hydroxy-dec-2-enoylamino)-2-hydroxymethyl-propyl ester represented by Chemical Formula 12. ¹H NMR analysis result of the white solid is as follows.

¹H NMR (300 MHz, CDCl₃) 6.83-6.50 (m, 2H), 6.21 (s, 1H), 5.70-5.50 (m, 2H), 4.29 (s, 2H), 4.18-4.14 (m, 2H), 3.69-3.65 (m, 2H), 3.53-3.49 (m, 6H), 2.4-1.80 (m, 26H).

### [Example 12] Preparation of 10-hydroxy-octadecanoic acid 3-hydroxy-2-hydroxymethyl-2-(10-hydroxy-octadecanoylamino)-propyl ester

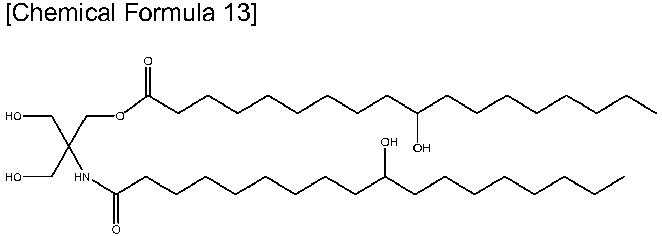

Tris(hydroxymethyl)aminomethane (10 g, 1 eq) was dissolved in dimethylformaldehyde (100 mL). After adding dicyclohexylcarbodiimide (34 g, 2 eq) and 4-dimethylaminopyridine (4.03 g, 0.2 eq), 10-hydroxystearic acid (49.5 g, 2 eq) was slowly added dropwise for 30 minutes while stirring at room temperature. After stirring for 6 hours, upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 14 g of white solid (39%) was obtained using a silica column. The obtained white solid was 10-hydroxy-octadecanoic acid 3-hydroxy-2-hydroxymethyl-2-(10-hydroxy-octadecanoylamino)-propyl ester represented by Chemical Formula 13. ¹H NMR analysis result of the white solid is as follows.

¹H NMR (300 MHz, CDCl₃) 6.21 (s, 1H), 4.29 (s, 2H), 4.18-4.14 (m, 2H), 3.69-3.65 (m, 2H), 3.60-3.53 (m, 2H), 3.53-3.49 (m, 2H), 2.37 (t, J = 7.5 Hz, 2H), 2.22 (t, J = 7.5 Hz, 2H), 1.62-1.11 (m, 54H), 0.90-0.86 (m, 6H).

### [Example 13] Preparation of 12-hydroxy-octadecanoic acid 3-hydroxy-2-hydroxymethyl-2-(12-hydroxy-octadecanoylamino)-propyl ester

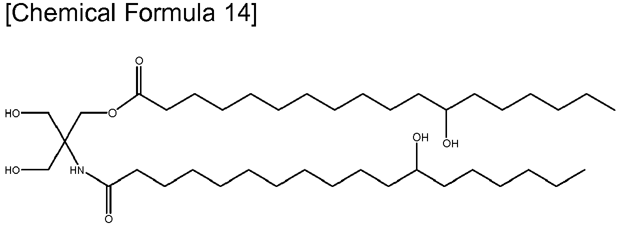

Tris(hydroxymethyl)aminomethane (10 g, 1 eq) was dissolved in dimethylformaldehyde (100 mL). After adding dicyclohexylcarbodiimide (34 g, 2 eq) and 4-dimethylaminopyridine (4.03 g, 0.2 eq), 12-hydroxystearic acid (49.5 g, 2 eq) was slowly added dropwise for 30 minutes while stirring at room temperature. After stirring for 6 hours, upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 12 g of white solid (32%) was obtained using a silica column. The obtained white solid was 12-hydroxy-octadecanoic acid 3-hydroxy-2-hydroxymethyl-2-(12-hydroxy-octadecanoylamino)-propyl ester represented by Chemical Formula 14. ¹H NMR analysis result of the white solid is as follows.

¹H NMR (300 MHz, CDCl₃) 6.21 (s, 1H), 4.29 (s, 2H), 4.18-4.14 (m, 2H), 3.69-3.65 (m, 2H), 3.60-3.52 (m, 2H), 3.53-3.48 (m, 2H), 2.37 (t, J = 7.5 Hz, 2H), 2.22 (t, J = 7.5 Hz, 2H), 1.62-1.11 (m, 54H), 0.90-0.86 (m, 6H).

### [Example 14] Preparation of 16-hydroxy-hexadecanoic acid 3-hydroxy-2-(16-hydroxy-hexadecanoylamino)-2-hydroxymethyl-propyl ester

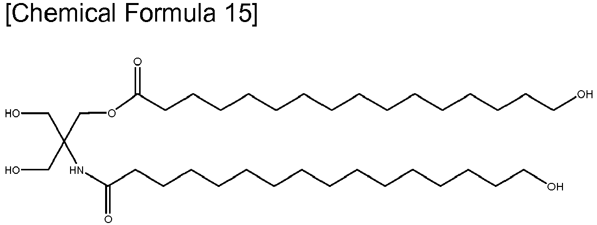

Tris(hydroxymethyl)aminomethane (10 g, 1 eq) was dissolved in dimethylformaldehyde (100 mL). After adding dicyclohexylcarbodiimide (34 g, 2 eq) and 4-dimethylaminopyridine (4.03 g, 0.2 eq), 16-hydroxydodecanoic acid (44.9 g, 2 eq) was slowly added dropwise for 30 minutes while stirring at room temperature. After stirring for 6 hours, upon completion of reaction, the mixture was diluted with ethyl acetate (200 mL) and washed with 1 N HCl solution (200 mL) and distilled water (200 mL). The organic layer was dried with anhydrous magnesium sulfate, filtered and concentrated under reduced pressure. Then, 17 g of white solid (45%) was obtained using a silica column. The obtained white solid was 16-hydroxy-hexadecanoic acid 3-hydroxy-2-(16-hydroxy-hexadecanoylamino)-2-hydroxymethyl-propyl ester represented by Chemical Formula 15. ¹H NMR analysis result of the white solid is as follows.

¹H NMR (300 MHz, CDCl₃) 6.21 (s, 1H), 4.29 (s, 2H), 4.18-4.14 (m, 2H), 3.69-3.64 (m, 6H), 3.60-3.52 (m, 2H), 3.53-3.48 (m, 2H), 2.37 (t, J = 7.5 Hz, 2H), 2.22 (t, J = 7.5 Hz, 2H), 1.60-1.54 (m, 12H), 1.30-1.20 (m, 40H).

### [Test Example 1] Recovery of damaged artificial skin (measurement of transepithelial electrical resistance)

Transepithelial electrical resistance was measured in order to test recovery of damaged artificial skin by the compound of Example 1. Dodecanoic acid 2-dodecanoylamino-3-hydroxy-2-hydroxymethyl-propyl ester obtained from the Example 1 and phosphate buffered saline (PBS), octyl methoxycinnamate (OMC) and PC-104 were tested for comparison.

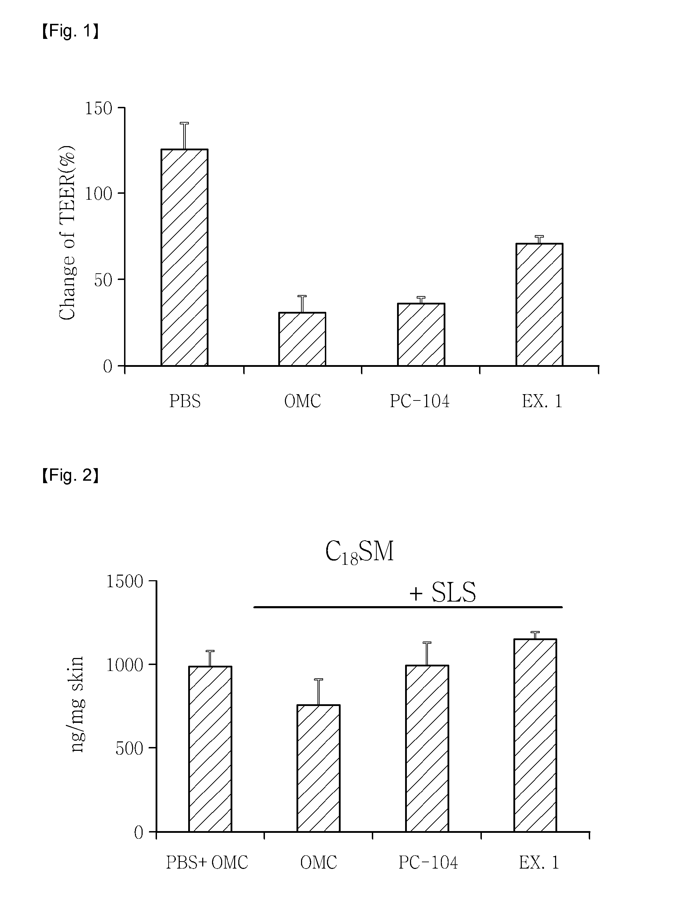

Transepithelial electrical resistance (TEER) of Keraskin™ purchased from MCTT was measured after 22 hours of pre-incubation. After placing the Keraskin™ on a 6-well plate, 400 µL of a medium was added in an insert and 5 mL of the medium was added outside the insert such that an even level was achieved inside and outside the insert. Then, measurement was made with the electrode tips of a resistance meter inside and outside the insert. TEER was measured for all the inserts, prior to the test, after treatment with the substances of Example 1 and Comparative Examples 1-3 and 24 hours after post-incubation. The TEER after the post-incubation was divided by the initial value to calculate the change in %. The result is shown in Table 1 and Fig. 1.

**[Table 1]**

| Test groups | Change of TEER (%) |
|---|---|
| Comparative Example 1 [phosphate buffered saline (PBS)] | 125.4 |
| Comparative Example 2 [octyl methoxycinnamate (OMC)] | 30.9 |
| Comparative Example 3 (PC-104) | 35.7 |
| Example 1 | 70.8 |

As can be seen from Table 1, when transepithelial electrical resistance was measured after treatment with the compound of Example 1, the effect of recovering damaged artificial skin was higher than the conventionally used PC-104.

### [Test Example 2] Quantitative analysis of ceramide in artificial skin

Artificial skin was weighed and put in a 4-mL glass vial. After adding 2400 µL of CHCl₃/MeOH (= 1/2) and 100 µL of IS STD, the artificial skin was cut into small pieces with scissors. After sonication for 30 minutes, solutions mixing sodium lauryl sulfate (SLS) to each of dodecanoic acid 2-dodecanoylamino-3-hydroxy-2-hydroxymethyl-propyl ester obtained from the Example 1, phosphate buffered saline (PBS), octyl methoxycinnamate (OMC) and PC-104, as described in Table 2, were filtered through a PTFE syringe filter, and 1250 µL of each filtrate was transferred to an EP tube.

After completely removing the solvent using a speed vacuum dryer, the residue was redissolved by adding 100 µL of an analytical mobile phase. After centrifugation, only the supernatant was taken and analyzed after transferring to an LC vial. The concentration of ceramide was corrected for the weight of the artificial skin. The result is shown in Table 2 and Fig. 2.

**[Table 2]**

| Test groups | C18SM | ng/mg skin |
|---|---|---|
| PBS + OMC | 1747.3 | 990.8 |
| SLS + OMC | 843.6 | 758.3 |
| SLS + PC-104 | 1141.1 | 995.5 |
| SLS + Example 1 | 1811.5 | 1152.4 |

As can be seen from Table 2, when the artificial skin was treated with the compound of Example 1, the ceramide content in the artificial skin was higher than that for PC-104. Accordingly, it can be concluded that the compound of Example 1 exhibits better moisturizing effect than PC-104 by increasing ceramides.

Hereinafter, formulation examples including the pseudo-ceramide compound according to the present disclosure, an isomer thereof, a pharmaceutically acceptable salt thereof, a prodrug thereof, a hydrate thereof or a solvate thereof will be described in detail. However, the following formulation examples are for illustrative purposes only and it will be apparent to those of ordinary skill in the art that the scope of the present disclosure is not limited by the examples.

### [Formulation Example 1] Lotion

Lotion was prepared according to a commonly employed method with the composition described in Table 3.

**[Table 3]**

| Ingredients | Contents (wt%) |
|---|---|
| Example 1 | 0.1 |
| Glycerin | 3.0 |
| Butylene glycol | 2.0 |
| Propylene glycol | 2.0 |
| Carboxyvinyl polymer | 0.1 |
| PEG 12 nonyl phenyl ether | 0.2 |
| Polysorbate 80 | 0.4 |
| Ethanol | 10.0 |
| Triethanolamine | 0.1 |
| Antiseptic, pigment and flavor | adequate |
| Purified water | balance |

### [Formulation Example 2] Nourishing cream

Nourishing cream was prepared according to a commonly employed method with the composition described in Table 4.

**[Table 4]**

| Ingredients | Contents (wt%) |
|---|---|
| Example 2 | 2.0 |
| Polysorbate 60 | 1.5 |
| Sorbitan sesquioleate | 0.5 |
| PEG 60 hydrogenated castor oil | 2.0 |
| Liquid paraffin | 10.0 |
| Squalane | 5.0 |
| Caprylic/capric triglyceride | 5.0 |
| Glycerin | 5.0 |
| Butylene glycol | 3.0 |
| Propylene glycol | 3.0 |
| Triethanolamine | 0.2 |
| Antiseptic, pigment and flavor | adequate |
| Purified water | balance |

### [Formulation Example 3] Massage cream

Massage cream was prepared according to a commonly employed method with the composition described in Table 5.

**[Table 5]**

| Ingredients | Contents (wt%) |
|---|---|
| Example 3 | 1.0 |
| Beeswax | 10.0 |
| Polysorbate 60 | 1.5 |
| PEG 60 hydrogenated castor oil | 2.0 |
| Sorbitan sesquioleate | 0.8 |
| Liquid paraffin | 40.0 |
| Squalane | 5.0 |
| Caprylic/capric triglyceride | 4.0 |
| Glycerin | 5.0 |
| Butylene glycol | 3.0 |
| Propylene glycol | 3.0 |
| Triethanolamine | 0.2 |
| Antiseptic, pigment and flavor | adequate |
| Purified water | balance |

### [Formulation Example 4] Pack

Pack was prepared according to a commonly employed method with the composition described in Table 6.

**[Table 6]**

| Ingredients | Contents (wt%) |
|---|---|
| Example 4 | 0.2 |
| Polyvinyl alcohol | 13.0 |
| Sodium carboxymethylcellulose | 0.2 |
| Glycerin | 5.0 |
| Allantoin | 0.1 |
| Ethanol | 6.0 |
| PEG 12 nonyl phenyl ether | 0.3 |
| Polysorbate 60 | 0.3 |
| Antiseptic, pigment and flavor | adequate |
| Purified water | balance |

### [Formulation Example 5] Gel

Gel was prepared according to a commonly employed method with the composition described in Table 7.

**[Table 7]**

| Ingredients | Contents (wt%) |
|---|---|
| Example 5 | 0.5 |
| Sodium ethylenediamineacetate | 0.05 |
| Glycerin | 5.0 |
| Carboxyvinyl polymer | 0.3 |
| Ethanol | 5.0 |
| PEG 60 hydrogenated castor oil | 0.5 |
| Triethanolamine | 0.3 |
| Antiseptic, pigment and flavor | adequate |
| Purified water | balance |

### [Formulation Example 6] Ointment

Ointment was prepared according to a commonly employed method with the composition described in Table 8.

**[Table 8]**

| Ingredients | Contents (wt%) |
|---|---|
| Example 6 | 1.5 |
| Glycerin | 8.0 |
| Butylene glycol | 4.0 |
| Liquid paraffin | 15.0 |
| β-Glucan | 7.0 |
| Carbomer | 0.1 |
| Caprylic/capric triglyceride | 3.0 |
| Squalane | 1.0 |
| Cetearyl glucoside | 1.5 |
| Sorbitan stearate | 0.4 |
| Cetearyl alcohol | 1.0 |
| Beeswax | 4.0 |
| Antiseptic, pigment and flavor | adequate |
| Purified water | balance |

## Claims

1. A novel pseudo-ceramide compound represented by Chemical Formula 1, an isomer thereof, a pharmaceutically acceptable salt thereof, a prodrug thereof, a hydrate thereof or a solvate thereof: 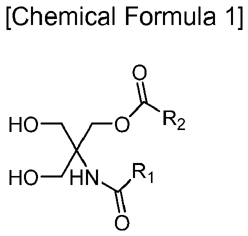 wherein each of R₁ and R₂ is independently a C₉-C₂₃ saturated or unsaturated aliphatic chain unsubstituted or substituted with a hydroxyl group.

2. The novel pseudo-ceramide compound, isomer thereof, pharmaceutically acceptable salt thereof, prodrug thereof, hydrate thereof or solvate thereof according to claim 1, wherein each of R₁ and R₂ is independently a C₁₁-C₁₇ saturated or unsaturated aliphatic chain unsubstituted or substituted with a hydroxyl group.

3. The novel pseudo-ceramide compound, isomer thereof, pharmaceutically acceptable salt thereof, prodrug thereof, hydrate thereof or solvate thereof according to claim 1, wherein each of R₁ and R₂ is independently selected from a group consisting of C₁₁H₂₃, C₁₃H₂₇, C₁₅H₃₁, C₁₇H₃₅, C₁₇H₃₁ and C₁₇H₃₃.

4. The novel pseudo-ceramide compound, isomer thereof, pharmaceutically acceptable salt thereof, prodrug thereof, hydrate thereof or solvate thereof according to claim 1, which is tris(hydroxymethyl)aminomethane substituted with a fatty acid.

5. The novel pseudo-ceramide compound, isomer thereof, pharmaceutically acceptable salt thereof, prodrug thereof, hydrate thereof or solvate thereof according to claim 1, which is selected from a group consisting of hexadecanoic acid 2-hexadecanoylamino-3-hydroxy-hydroxymethyl-propyl ester, dodecanoic acid 2-dodecanoylamino-3-hydroxy-2-hydroxymethyl-propyl ester, tetradecanoic acid 3-hydroxy-2-hydroxymethyl-2-tetradecanoylamino-propyl ester, octadecanoic acid 3-hydroxy-2-hydroxymethyl-2-octadecanoylamino-propyl ester, octadecanoic acid 2-dodecanoylamino-3-hydroxy-2-hydroxymethyl-propyl ester, octadecanoic acid 3-hydroxy-2-hydroxymethyl-2-tetradecanoylamino-propyl ester, tetradecanoic acid 3-hydroxy-2-hydroxymethyl-2-octadecanoylamino-propyl ester, dodecanoic acid 3-hydroxy-2-hydroxymethyl-2-octadecanoylamino-propyl ester, octadec-9-enoic acid 3-hydroxy-2-hydroxymethyl-2-octadec-9-enoylamino-propyl ester, octadeca-9,12-dienoic acid 3-hydroxy-2-hydroxymethyl-2-octadeca-9,12-dienoylamino-propyl ester, 10-hydroxy-dec-2-enoic acid 3-hydroxy-2-(10-hydroxy-dec-2-enoylamino)-2-hydroxymethyl-propyl ester, 10-hydroxy-octadecanoic acid 3-hydroxy-2-hydroxymethyl-2-(10-hydroxy-octadecanoylamino)-propyl ester, 12-hydroxy-octadecanoic acid 3-hydroxy-2-hydroxymethyl-2-(12-hydroxy-octadecanoylamino)-propyl ester and 16-hydroxy-hexadecanoic acid 3-hydroxy-2-(16-hydroxy-hexadecanoylamino)-2-hydroxymethyl-propyl ester.

6. A method for preparing the novel pseudo-ceramide compound, isomer thereof, pharmaceutically acceptable salt thereof, prodrug thereof, hydrate thereof or solvate thereof according to any one of claims 1 to 5, comprising reacting tris(hydroxymethyl)aminomethane and a fatty acid compound under a basic condition to synthesize a pseudo-ceramide compound.

7. The method for preparing the novel pseudo-ceramide compound, isomer thereof, pharmaceutically acceptable salt thereof, prodrug thereof, hydrate thereof or solvate thereof according to claim 6, wherein the method is represented by Scheme 1: 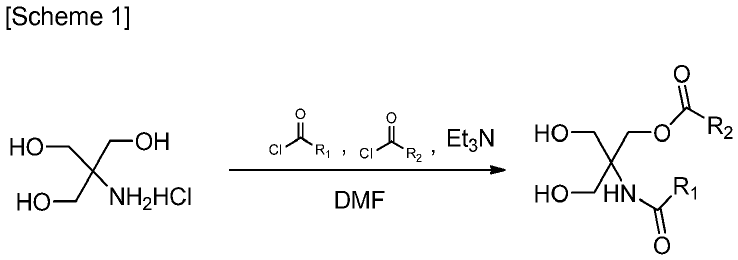 wherein each of R₁ and R₂ is independently a C₉-C₂₃ saturated or unsaturated aliphatic chain unsubstituted or substituted with a hydroxyl group.

8. The method for preparing the novel pseudo-ceramide compound, isomer thereof, pharmaceutically acceptable salt thereof, prodrug thereof, hydrate thereof or solvate thereof according to claim 7, wherein each of R₁ and R₂ is independently a C₁₁-C₁₇ saturated or unsaturated aliphatic chain unsubstituted or substituted with a hydroxyl group.

9. The method for preparing the novel pseudo-ceramide compound, isomer thereof, pharmaceutically acceptable salt thereof, prodrug thereof, hydrate thereof or solvate thereof according to claim 7, wherein each of R₁ and R₂ is independently selected from a group consisting of C₁₁H₂₃, C₁₃H₂₇, C₁₅H₃₁, C₁₇H₃₅, C₁₇H₃₁ and C₁₇H₃₃.

10. A composition for moisturizing skin, comprising the novel pseudo-ceramide compound, isomer thereof, pharmaceutically acceptable salt thereof, prodrug thereof, hydrate thereof or solvate thereof according to any one of claims 1 to 5 as an active ingredient.

11. The composition for moisturizing skin according to claim 10, wherein the active ingredient is included in an amount of 0.01-20 wt% based on the total weight of the composition.

12. The composition for moisturizing skin according to claim 10, wherein the composition is a composition for external application to skin.

13. The composition for moisturizing skin according to claim 10, wherein the composition is a cosmetic composition or a pharmaceutical composition.
